# EUROPEAN PATENT APPLICATION

(11) **EP 4 835 095 A2**
(43) Date of publication of application: **04.11.2026**
(21) Application number: 26190546.7
(22) Date of filing: 17.05.2022
(51) Int. Cl.: C12Q 1/6844

(54) **PERTURBED GENOMIC EXPRESSION IN PRETEMPLATED INSTANT PARTITIONS**

(30) Priority: 18.05.2021 US 202163190125 P
(62) Document number(s) of the earlier application(s) in accordance with Art. 76 EPC: 22805347.6 / 4 340 947
(71) Applicant: Illumina, Inc., San Diego, CA 92122 (US)
(72) Inventor: FONTANEZ, Kristina, Arlington, MA 02476 (US); CLARK, Iain, Watertown, MA 02472 (US); D'AMATO, Christopher, Wellesley, MA 02481 (US); KUGLER, Catherine, Watertown, MA 02472 (US); MELTZER, Robert, Belmont, MA 02478 (US)
(74) Representative: Robinson, David Edward Ashdown

(57) **Abstract**

This invention provides methods for near-instantaneously separating cells that have undergone RNA guided genome modifications into pre-templated instant partitions (PIPs) and using the PIPs to associate the guide RNAs with the gene expression level changes that resulted from the genome modification.

## Description

### Technical Field

The invention generally relates to the field of genomic expression capture and sequencing.

### Background

Cancer is the second leading cause of death globally, responsible for nearly ten million deaths every year. Cancer is a genetic disease, caused by hereditary or acquired mutations in genetic information stored in DNA that control how cells grow and divide. There are many different kinds of mutations in DNA that have been linked with cancers, but generally each individual cancer patient's profile of genetic mutations is different. Moreover, the genetic information stored in DNA must first be transcribed into messenger RNA (mRNA) before being expressed in a cell. The degree to which genetic information in DNA is actually expressed into mRNA is also unique to each individual. That is to say, even if a patient can be identified as having a DNA mutation associated with cancer, it would remain unknown whether and to what degree the patient may actually express a cancer phenotype.

Single-cell sequencing of RNA has revolutionized the detection of cancer cell expression in patients by providing scientists with the actual expression levels of genes associated with cancers in cells from the complex mixtures from which they are prepared. Popular methods for isolating single cells and their RNA expression employ flow cytometry and droplet microfluidics to separate single cells one at a time. These methods, however, require complicated equipment that is both expensive and difficult to use. Moreover, because each cell must be processed individually, such methods are rate limited and require extensive periods of time (often days) to separate cancer cells from surrounding cells. This limitation is especially problematic in early cancer detection, where the proportion of cancer cells to a sample is at its smallest. Additionally, because such methods are difficult to use, particularly by clinicians, samples must be sent to facilities capable of handling such equipment, further increasing the time and expense needed to reach a cancer diagnosis. This has resulted in early detection of cancer gene expression being unaffordable and unavailable to the majority of cancer patients.

### Summary

This invention provides methods for single cell gene expression analysis that greatly reduce the complexity and cost of gene expression sequencing and cancer detection. The present invention provides methods for near-instantaneously separating cells that have undergone RNA guided genome modifications into pre-templated instant partitions (PIPs) and using the PIPs to associate the guide RNAs with the gene expression level changes that resulted from the genome modification.

Advantageously, each cell comprises copies of the RNA guide that modified the cell. For example, each cell may continue to express copies of the RNA guide. As a result, both the mRNA expressed by the cells and the RNA guide can be barcoded with a sequence unique to the partition. Each RNA guide can then be associated with the mRNA transcripts actually expressed by the cell as a result of the genomic modification caused by that RNA guide. By partitioning genetically modified cells simultaneously, the invention allows for the evaluation of thousands or hundreds of thousands of genetic modifications in cells within days, rather than weeks. Additionally, methods of the present invention are performed without the need for complex and expensive machinery as required by microfluidic cell separation techniques, dramatically reducing the cost of gene expression analysis and profiling in early cancer detection.

The instant invention describes a method for gene expression profiling that comprises combining a plurality of cells with template particles. The cells have been genetically modified by an RNA guided endonuclease and the cells comprise a copy of the RNA guide that further expresses a capture sequence. The copy of the RNA guide is a copy of the same guide that together with the RNA guided endonuclease genetically modified the cell. A plurality of uniform partitions are generated near-instantly that encapsulate a single one of the template particles and a single one of the cells to form pre-templated instant partitions (PIPs). Nucleic acid molecules are released in each PIP from the cells, including at least the RNA guides that comprise a capture sequence and RNA transcripts. Advantageously, the steps of combining the cells and template particles and forming a plurality of uniform partitions may be performed in the same container, for example a tube, for example a centrifuge tube. Moreover, the cells may continue to express the RNA guide after genetic modification by the guide.

Once RNA is released from cells, the RNA may be reverse transcribed into cDNA compliments of the RNA. This is advantageous, because cDNA is generally more stable than RNA and more easily available and processed for sequencing. Moreover, the reverse transcription reaction to cDNA is useful for further adding a unique molecular identifier and a barcode unique to the PIP to each nucleic acid molecule.

Generally, reverse transcription is performed by oligo dT reverse transcription primers that hybridize to the poly adenylated (polyA) tail of RNA. Accordingly, the mRNA transcripts may be reverse transcribed by using oligo dT reverse transcription primers. However, oligo dT reverse transcription primers are generally more limited in reverse transcribing non-polyadenylated RNA.

Non-polyadenylated copies of RNA guides are generally needed for RNA guided platforms, because the polyA tail of RNA interferes with the function of the RNA guided platforms.

Advantageously, in the present invention, non-polyadenylated RNA guides may comprise a capture sequence and the step of reverse transcribing the non-polyadenylated RNA guides may comprise hybridizing a capture primer for reverse transcription to the capture sequence of the non-polyadenylated RNA guides. Further advantageously, the capture sequence on the RNA guide may be located in a region that minimally interferes with the gene editing function of the guide. For example, the RNA guides may comprise constant regions, for example a stem loop 2 or 3' end region, that minimally affects the activity of the guide RNA when altered. RNA guides comprising a capture sequence in the stem loop 2 or 3' region are useful when a 3' sequence library may be desired to sequence the cDNA.

The non-polyadenylated RNA guide may also itself comprise the unique molecular identifier. The advantage of including the unique molecular identifier on the RNA guide, is that the capture primer for reverse transcription can be unbarcoded, which will not comprise the unique molecular identifier. Reverse transcribing the mRNA transcripts may then comprise using, for example, an oligo dT reverse transcription primer to form the cDNA and add the unique molecular identifier and barcode unique to the PIP to the cDNA. In contrast, reverse transcribing the RNA guides that have a unique molecular identifier may comprise a capture primer for reverse transcription that adds only the barcode unique to the PIP and not the unique molecular identifier. RNA guides comprising a unique molecular identifier and unbarcoded reverse transcription primers may comprise a capture sequence towards the 5' end of the RNA guide. RNA guides comprising a unique molecular identifier are useful when a 5' sequence library may be desired to sequence the cDNA.

The invention also describes a method for gene expression profiling comprising combining a plurality of cells with template particles. The cells have been genetically modified by an RNA guided endonuclease and the cells comprise a non-polyadenylated copy of the RNA guide that together with the RNA guided endonuclease modified the genome of the cell. Advantageously, the cells also comprise a polyadenylated RNA molecule that comprises a guide barcode associated with the guide RNA. A plurality of uniform partitions are generated near-instantly that encapsulate a single one of the template particles and a single one of the cells to form pre-templated instant partitions (PIPs). Nucleic acid molecules are released in each PIP from the cells, including at least the polyadenylated RNA molecules comprising the guide barcode and RNA transcripts. Because both the RNA molecule comprising the guide barcode and RNA transcripts are polyadenylated, a poly T reverse transcription primer that hybridizes to the polyA tail of RNA can be used to reverse transcribe the RNA molecules.

The advantage of reverse transcribing cDNA is that cDNA is generally more stable than RNA and more easily available and processed for sequencing. After the reverse transcription step, the cDNA molecules may be amplified, for example by a polymerization reaction, to form amplicons. The cDNA or amplicons may also be sequenced to generate sequence reads associated with each RNA guide and sequence reads associated with each RNA transcript. For example, the sequence read associated with each RNA guide may be from sequencing cDNA from a non-polyadenylated RNA guide when a capture sequence is used to reverse transcribe the RNA guide. The sequence read associated with each RNA guide may be a sequence read from sequencing cDNA from the RNA molecules comprising the guide barcode associated with the RNA guide.

Once sequenced, each RNA guide sequence read can be associated with each RNA transcript sequence read having the same barcode unique to a PIP. Any change in the expression of RNA transcripts can then be associated with the RNA guide. As a result, changes to the genome effected by the RNA guide can be evaluated for the actual changes in gene expression that result. As a result, the methods of the invention provide for genetic analysis that greatly reduce the complexity and cost of evaluating phenotypic changes that result from genotypic changes.

Moreover, because each RNA molecule comprises a unique molecular identifier (UMI) not only can the number of different RNA transcripts that are expressed be evaluated, but also the quantity of each mRNA transcript. This is because UMIs are a type of barcode that together with the nucleic acid molecule that they are tagged to, make each nucleic acid molecule unique or nearly unique. This is accomplished by adding, e.g. by reverse transcription, a UMI to each nucleic acid molecule such that it is unlikely that any two previously identical nucleic acid molecules, together with their UMIs, have the same sequence. By selecting an appropriate number of UMIs, every nucleic acid molecule in the sample, together with its UMI, will be unique or nearly unique. For example, where 100 identical copies of an mRNA transcript associated with cancer are expressed, each copy together with its UMI will be made unique. After several rounds of amplification, each now unique copy may be differentially amplified multiple times, so that thousands of copies of the mRNA transcript are available for sequencing. Once sequenced, by mapping or counting (for example, by collapsing reads) only fully unique sequences, the original count of 100 expressed mRNA transcripts can be discerned.

In aspects of the invention, a plurality of cells may be modified by a plurality of guide RNAs that are each associated with a different genetic modification associated with a disease, for example cancer. Because each guide RNA is associated with the genetic modification and each guide RNA is also associated with the gene transcripts in a PIP resulting from that modification, each modification can be analyzed for the phenotypic changes that result from the genetic modification.

Any RNA guided platform may be useful with the instant invention, for example an RNA guided platform using an RNA guided endonuclease, for example a Cas endonuclease.

The present invention is based on the partitioning of cells near-instantly, rather than one by one, into pre-templated instant partitions (PIPs).

Methods of the present invention may simultaneously separate single cells by combining the template particles with the single cells in a first fluid, adding a second fluid to the first fluid, and agitating the fluids to generate a plurality of pre-templated instant partitions simultaneously that contain a single one of the template particles and a single one of the single cells. Simultaneously or near-instantly are used interchangeably and include within seconds, within
minutes, or within hours. Simultaneously or near-instantly does not include in serial as envisioned by microfluidic equipment.

The first fluid and the second fluid may be immiscible. For example, the first fluid may comprise an aqueous phase fluid and/or the second fluid may comprise an oil. The first fluid may comprise reagents selected from, for example, buffers, salts, lytic enzymes (e.g. proteinase k) and/or other lytic reagents (e. g. Triton X-100, Tween-20, IGEPAL, or combinations thereof), nucleic acid synthesis reagents e.g. nucleic acid amplification reagents or reverse transcription mix, or combinations thereof. The second fluid may comprise fluorocarbon oil, a silicone oil, or a hydrocarbon oil, or a combination thereof. Agitating fluids may comprise vortexing, shaking, flicking, stirring, pipetting, or any known method for mixing solutions.

The step of releasing in each PIP nucleic acid molecules from the cells may comprise lysing each of the single cells contained within the PIPs. Releasing nucleic acid molecules or proteins from single cells may comprise lysis of the single cells within the PIPs. Lysis may be induced by a stimulus such as heat, osmotic pressure, lytic reagents (e.g., DTT, betamercaptoethanol), detergents (e.g., SDS, Triton X-100, Tween-20), enzymes (e.g., proteinase K), or combinations thereof.

Template particles may comprise any known particles that can be useful for forming PIPs. The template particles may be hydrogels, for example, hydrogels comprising agarose, alginate, a polyethylene glycol (PEG), a polyacrylamide (PAA), acrylate, acrylamide/bis-acrylamide copolymer matrix, azide-modified PEG, poly-lysine, polyethyleneimine, and combinations thereof. In certain instances, template particles may be shaped to provide an enhanced affinity for the single cells. For example, the template particles may be generally spherical but the shape may contain features such as flat surfaces, craters, grooves, protrusions, and other irregularities in the spherical shape that promote an association with a single cell such that the shape of the template particle increases the probability of templating a PIP that contains a single cell. Tubes for single cell analysis of the present invention may be selected based on the volume of sample from which cells need to be separated and/or based on the number of cells to be separated. For example, the tube may be a single large tube, such as a conical centrifuge tube, such as those sold under the trade name FALCON as sold by Corning Inc., Corning, New York, for example a tube with a volume of less than 40 mL. The tubes may also be wells, such as standard 96 sample well kits. The tubes may also be centrifuge, microcentrifuge, or PCR tubes, such as those sold under the trade name EPPENDORF as sold by Eppendorf, Hamburg, Germany. Such tubes, for example, may be between .1 and 6mL.

The step of generating in the tube a plurality of uniform partitions near-instantly that encapsulate a single one of the template particles and a single one of the cells may also comprise generating a plurality of partitions that encapsulate a single one of the template particles and do not encapsulate a cell. For example, where 100,000 template particles are combined with 10,000 single cells in a first fluid, it is expected that generating partitions may result in about 90,000 partitions that encapsulate a single template particle and do not encapsulate a cell. Additionally in rarer cases, two template particles may be encapsulated by a single partition, or two cells may be encapsulated in a single partition.

### Brief Description of the Drawings

FIG. 1 diagrams aspect of methods of the invention.
FIG. 2 diagrams an aspect of methods of the invention.
FIG. 3 shows a graph of cDNA fractions following reverse transcription.

### Detailed Description

The present invention provides methods for near-instantaneously separating cells that have undergone RNA guided genome modifications into pre-templated instant partitions (PIPs) and using the PIPs to associate the guide RNAs with the gene expression level changes that resulted from the genome modification.

The present invention provides methods for reverse transcribing RNA guides to generate cDNA molecules from the RNA guides that are tagged with a UMI and barcode unique to a PIP. mRNA transcripts from the PIP, and therefore a single cell, are also tagged with a UMI and barcode unique to the PIP. The cDNA can then be amplified to generate amplicons and analyzed, for example by sequencing. Methods for analyzing cells modified by RNA guides are described in Dixit, 2016, Cell 1687:1853-1866, and Replogle, 2020, Nature 38:954-961, the entirety of each of which are incorporated herein by reference.

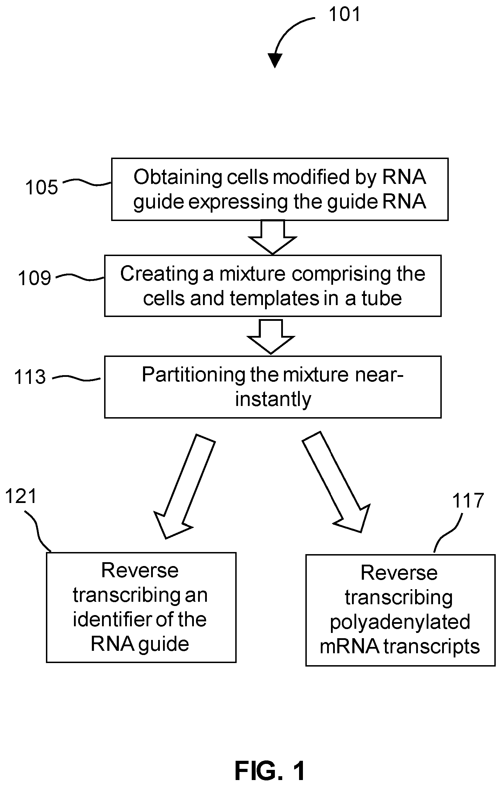

FIG. 1 diagrams methods of the invention generally. The method 101 includes obtaining cells that have been modified by an RNA guided editing platform expressing an RNA guide 105. The cells are mixed in a tube 109 with template particles and partitioned 113 to create a plurality of partitions encapsulating one of the cells 105 and one template particle. An RNA identifier of the RNA guide is reverse transcribed 121 into cDNA in parallel with reverse transcription of polyadenylated mRNA transcripts 117.

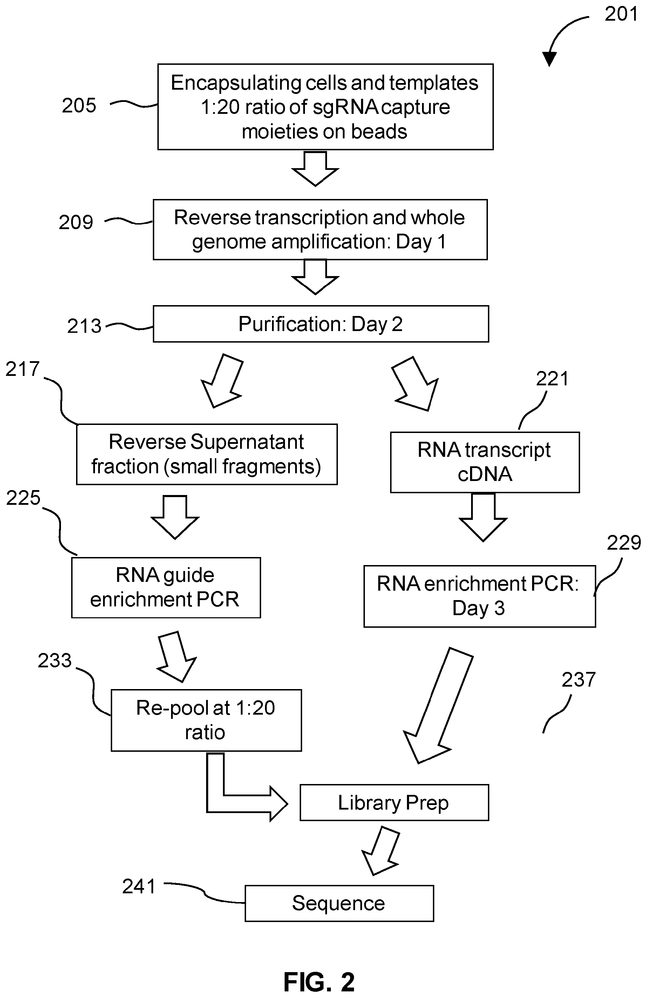

FIG. 2 diagrams a detailed method of the invention. The method 201 includes encapsulating cells and templates 205 along with capture primers for reverse transcription of the RNA guides comprising a capture sequence and primers for reverse transcription of mRNA transcripts, for example oligo dT, at a ratio of 1:20 capture primers to oligo dT. Reverse transcription is initiated to generate cDNA from the RNA guides and cDNA from the mRNA transcripts and amplified by whole genome amplification 209, with partitioning of cells 205, reverse transcription and amplification taking place in a single day. During Day 2, the cDNA is then purified 213, for example by centrifugation, with the smallest fragments in the supernatant representing the cDNA generated from the RNA guides 217 with the precipitate containing cDNA generated from RNA transcripts 221. During Day 3 cDNA from RNA guides 225 and cDNA from RNA transcripts are enriched 229 in parallel. The cDNA from RNA transcripts are prepared for sequencing library prep 237 and the cDNA from RNA guides is re-pooled 233 with the library prepped mRNA at a 1:20 ratio and the cDNA is sequenced 241.

### Gene expression profiling and genetic disease

Gene expression profiling is the measurement of the actual expression of genes. Gene expression profiling includes the identification of mRNA being expressed and the measurement of the quantity of that mRNA in the cell to measure the activity of the corresponding genes. While sequencing a genome provides information as to what the cell could possibly do, the expression profile provides information as to what the cell is actually doing at a point in time.

At any moment, generally each cell makes mRNA from only a fraction of the genes it carries. If a gene is used to produce mRNA, it is generally considered "on", otherwise "off" For example, cells may be modified by an RNA guide that is thought to produce an "on" switch in a gene, an RNA guide that is thought to produce as "off" switch in a gene, and an RNA guide that is thought to produce no change in the gene. The gene expression profile provides information as to what the changes made by the guide RNAs in DNA actually result in phenotypic changes in the mRNA expression in the cell. Gene expression profiling may also provide information as to the editing capacity of an RNA guide. For example, when multiple RNA guides targeting the same "on" switch are analyzed in parallel, the varying levels of gene expression changes may be used to analyze the activity of the guide.

Gene expression profiling is useful for analyzing genetic diseases with varying disease states, for example cancers, neurodegenerative diseases, neuropsychiatric disease, metabolic disorders, and cardiovascular disorders. Metabolic disorders may include type 2 diabetes and obesity. Cardiovascular disorders may include atherosclerosis and hypertension. Neurological disorders may include Alzheimer's or Parkinson's. Cancers may include Hodgkin lymphoma, non-Hodgkin lymphoma, myelodysplastic syndromes, breast cancer, prostate cancer, melanoma, ovarian cancer, sarcoma, oral carcinoma, or a hepatocellular carcinoma.

Moreover, gene expression profiling can be useful for identifying the mechanisms of action of therapeutic interventions, for example small molecule drugs.

### RNA guided gene editing

An endonuclease is an enzyme that cleaves the phosphodiester bond within a polynucleotide gene, for example genomic DNA. Endonucleases can cleave DNA relatively non-specifically or at very specific nucleotide sequences. RNA guided endonucleases are endonucleases that use an RNA guide to target a location on DNA. In this way, the RNA guide or guide RNA, used herein interchangeably, confers target sequence specificity to the RNA guided endonuclease.

Cas9 (Clustered Regularly Interspaced Short Palindromic Repeats (CRISPR) associated protein 9) is an RNA guided endonuclease that plays a role in the immunological defense of certain bacteria against DNA viruses. CRISPR-Cas9 is a gene editing tool that is a dual-RNA guided DNA endonuclease. Cas9 generally interrogates DNA by checking for sites complementary to a 20 base pair spacer region on an RNA guide. If the DNA is complementary to the guide RNA, Cas9 cleaves the DNA. Advantageously, Cas9 can cleave nearly any sequence complementary to the guide RNA. Cas9 is considered a dual-RNA guided DNA endonuclease because native Cas9 requires a guide RNA composed of two RNAs that associate, a CRISPR RNA (crRNA) and a trans-activating crRNA (tracrRNA). Cas9 targeting is frequently simplified by using a single guide RNA (sgRNA) that combines the crRNA and tracrRNA into a single RNA molecule.

RNA guides useful with the methods of the invention may comprise crRNAs, tracrRNAs, guide RNA spacers, and sgRNAs.

Typically, CRISPR-Cas9 targeting specificity is determined by the 20 base pair sequence at the 5' end of the RNA guide. The desired target sequence must precede a protospacer adjacent motif, which is a short DNA sequence typically 2-6 base pairs in length that is typically 3-4 nucleotides downstream of the DNA region targeted for modification. After base pairing of the RNA guide to the target, Cas9 mediates a double strand break about 3 base pairs upstream of the protospacer adjacent motif,

RNA guided endonucleases may introduce gene knock out or knock in depending on the double strand repair pathway. RNA guided endonucleases may also upregulate expression of a gene or gene transcripts by knocking out a repressor gene or downregulate expression of a gene or gene transcript by knocking out a promoter of the gene.

### Barcodes and Unique Molecular Identifiers

Barcodes specific to each PIP may be any group of nucleotides or oligonucleotide sequences that are distinguishable from other barcodes within the group. A PIP encapsulating a template particle and a single cell provides to each nucleic acid molecule released from the single cell the same barcode from the group of barcodes. The barcodes provided by each PIP are unique to that PIP and distinguishable from the barcodes provided to nucleic acid molecules by every other PIP. Once sequenced, by using the barcode sequence, the nucleic acid molecules can be traced back to the PIP and thereby to each single cell. Barcodes may be of any suitable length sufficient to distinguish the barcode from other barcodes. For example, a barcode may have a length of 4, 5, 6, 7, 8, 9, 10, 11, 12, 13, 14, 15, 16, 17, 18, 19, 20, 21, 22, 23, 24, 25 nucleotides, or more.

The barcodes unique to each PIP may be pre-defined, degenerate, and/or selected at random. Barcodes may be added to nucleic acid molecules by "tagging" the nucleic acid molecules with the barcode. Tagging may be performed using any known method for barcode addition, for example direct ligation of barcodes to one or more of the ends of each nucleic acid molecule. Nucleic acid molecules may, for example, be end repaired in order to allow for direct or blunt-ended ligation of the barcodes. Barcodes may also be added to nucleic acid molecules through first or second strand synthesis, for example using capture probes or primers.

Unique molecular identifiers are a type of barcode that may be provided to nucleic acid molecules in a sample to make each nucleic acid molecule, together with its barcode, unique, or nearly unique. This is accomplished by adding, e.g. by ligation, one or more UMIs to each nucleic acid molecule such that it is unlikely that any two previously identical nucleic acid molecules, together with their UMIs, have the same sequence. By selecting an appropriate number of UMIs, every nucleic acid molecule in the sample, together with its UMI, will be unique or nearly unique. One strategy for doing so is to provide to a sample of nucleic acid molecules a number of UMIs in excess of the number of starting nucleic acid molecules in the sample. By doing so, each starting nucleic molecule will be provided with different UMIs, therefore making each molecule together with its UMIs unique. However, the number of UMIs provided may be as few as the number of identical nucleic acid molecules in the original sample. For example, where no more than six nucleic acid molecules in a sample are likely to be identical, as few as six different UMIs may be provided, regardless of the number of starting nucleic acid molecules.

UMIs are also advantageous in that they can be useful to correct for errors created during amplification, such as amplification bias or incorrect base pairing during amplification. For example, when using UMIs, because every nucleic acid molecule in a sample together with its UMI or UMIs is unique or nearly unique, after amplification and sequencing, molecules with identical sequences may be considered to refer to the same starting nucleic acid molecule, thereby reducing amplification bias. Methods for error correction using UMIs are described in Karlsson et al., 2016, "Counting Molecules in cell-free DNA and single cells RNA", Karolinska Institutet, Stockholm Sweden, the contests of which are incorporated herein by reference.

### Template particles

The template particles of the present disclosure may be prepared using any method known in the art. Generally, the template particles are prepared by combining hydrogel material, e.g., agarose, alginate, a polyethylene glycol (PEG), a polyacrylamide (PAA), Acrylate, Acrylamide/bisacrylamide copolymer matrix, and combinations thereof. Following the formation of the template particles they are sized to the desired diameter for capturing and uniquely tagging cells. For example, sizing of the template particles may be done by microfluidic co-flow into an immiscible oil phase.

Template particles may vary in size. Variation may be limited, for example the diameter or largest dimension of the template particles may be such that at least 50% or more, e.g., 60% or more, 70% or more, 80% or more, 90% or more, 95% or more, or 99% or more of the template particles vary in diameter or largest dimension by less than a factor of 10, e.g., less than a factor of 5, less than a factor of 4, less than a factor of 3, less than a factor of 2, less than a factor of 1.5, less than a factor of 1.4, less than a factor of 1.3, less than a factor of 1.2, less than a factor of 1.1, less than a factor of 1.05, or less than a factor of 1.01.

Advantageously, the absorbency of the presently disclosed template particles may be increased by storing them in a dehydrated condition prior to using them in the presently disclosed method for single cell analysis, with the general intention of shrinking their volume. Advantageously, shrinking template particles allows for control of the template particle shape and size for capturing cells and barcoding released nucleic acid molecules, for example with barcodes unique to each PIP. For example, dehydration of the template particles may be achieved by storing them in a high osmolarity buffer to promote shrinking (i.e. Polyethelene glycol). Alternatively, the template particles may be ethanol dehydrated. Shrinking may occur upon the application of an external stimulus, e.g., heat. For instance, the template particles may be encapsulated in a fluid by shearing, followed by the application of heat, causing the template particles to shrink in size. Some other examples of drying approaches include, but are not limited to, heating, drying under vacuum, freeze drying, and supercritical drying. The dried template particles may also be combined with a fluid, but still retain the shape and structure as independent, often spherical, gel particles. The dried template particles may be combined with an appropriate fluid, causing a portion of the fluid to be absorbed by the template particles. Porosity of the template particles may also vary, to allow at least one of a plurality of cells to be absorbed into the template particles when combined with the appropriate fluid. Any convenient fluid that allows for the desired absorption to be performed in the template particles may be useful for methods of the invention.

Template particles are advantageously tiny, generally spherical, particles. Template particles may be porous or nonporous. Template particles may also include microcompartments or internal compartments which advantageously may contain additional components and/or reagents, for example, additional components and/or reagents that may be releasable into PIPs.

Template particles may include a polymer such as a hydrogel. Template particles generally range from about 0.1 to about 1000 µm in diameter or largest dimension. Template particles may have a diameter or largest dimension of about 1.0 µm to 1000 µm, inclusive, such as 1.0 µm to 750 µm, 1.0 µm to 500 µm, 1.0 µm to 250 µm, 1.0 µm to 200 µm, 1.0 µm to 150 µm 1.0 µm to 100 µm, 1.0µm to 10 µm, or 1.0 µm to 5 µm, inclusive. Template particles may have a diameter or largest dimension of about 10 µm to about 200 µm, e.g., about 10 µm to about 150 µm, about 10 µm to about 125 µm, or about 10 µm to about 100 µm.

Cells analyzed by the present invention may include live cells obtained from, for example, a sample (tissue of bodily fluid) of a patient. The sample may include a fine needle aspirate, a biopsy, or a bodily fluid from the patient. Upon being isolated from the sample, the cells may be processed by, for example, generating a single cell suspension with an appropriate solution. Such solution will generally be a balanced salt solution, e.g. normal saline, PBS, HBSS (Hank's balanced salt solution), etc., and in certain instances supplemented with fetal calf serum or other naturally occurring factors, in conjunction with an acceptable buffer at low concentration, generally from 5-25 mM. Convenient buffers include HEPES, phosphate buffers, lactate buffers, etc. The separated cells can be collected in any appropriate medium that maintains the viability of the cells, usually having a cushion of serum at the bottom of the collection tube. Various media are commercially available and may be useful according to the nature of the cells, including dMEM, HBSS, DPBS, RPMI, IMDM (Iscove's medium), etc., frequently supplemented with fetal calf serum. Preferably, the cells are mammal cells, for example human cells.

The composition and nature of the template particles may vary depending on the single cell analysis being conducted. For instance, the template particles may be microgel particles that are micron-scale spheres of gel matrix. The microgels are composed of a hydrophilic polymer that is soluble in water, including alginate or agarose. Microgels may also be composed of a lipophilic microgel.

Template particles may also be a hydrogel, such as hydrogels from naturally derived materials, synthetically derived materials, and combinations thereof. Examples of hydrogels include, but are not limited to, collagen, hyaluronan, chitosan, fibrin, gelatin, alginate, agarose, chondroitin sulfate, polyacrylamide, polyethylene glycol (PEG), polyvinyl alcohol (PVA), acrylamide/bisacrylamide copolymer matrix, polyacrylamide /poly(acrylic acid) (PAA), hydroxyethyl methacrylate (HEMA), poly N- isopropylacrylamide (NIPAM), and polyanhydrides, poly(propylene fumarate) (PPF).

Template particles may further advantageously comprise materials which provide the template particles with a positive surface charge, or an increased positive surface charge. Such materials may be without limitation poly-lysine or Polyethyleneimine, or combinations thereof. This may increase the chances of association between the template particle and, for example, a cell which generally have a mostly negatively charged membrane.

Other strategies aimed to increase the chances of template particle-cell association include creation of a specific template particle geometry. For example, the template particles may have a general spherical shape but the shape may contain features such as flat surfaces, craters, grooves, protrusions, and other irregularities in the spherical shape.

Any one of the above described strategies and methods, or combinations thereof may be useful in the practice of the presently disclosed template particles and method for single cell analysis thereof. Methods for generation of template particles, and template particles-based encapsulations, were described in International Patent Publication WO 2019/139650, which is incorporated herein by reference.

To increase the chances of generating partitions that contain one template particle and one single cell, the template particles and cells may be combined at a ratio wherein there are more template particles than cells. For example, the ratio of template particles to cells combined in a mixture as described above may be in a range of 5:1 to 1,000:1, respectively. The template particles and cells may also be combined at a ratio of 10:1, 100: 1, or 1000:1, respectively.

To generate a monodisperse emulsion, a step of shearing the second mixture provided by combining a first mixture comprising template particles and cells with a second fluid immiscible with the first mixture. Any suitable method may apply a sufficient shear force to the second mixture. For example, the second mixture may be sheared by flowing the second mixture through a pipette tip. Other methods include, but are not limited to, shaking the second mixture with a homogenizer (e.g., vortexer), or shaking the second mixture with a bead beater. Vortexing may be performed for example for 30 seconds, or in the range of 30 seconds to 5 minutes. The application of a sufficient shear force breaks the second mixture into partitions that encapsulate one of a plurality of template particles.

Generating the template particle-based partitions may involve shearing two liquid phases. For example, the mixture may be the aqueous phase and comprise reagents selected from, for example, buffers, salts, lytic enzymes (e.g. proteinase k) and/or other lytic reagents (e. g. Triton X-100, Tween-20, IGEPAL, bm 135, or combinations thereof), nucleic acid synthesis reagents e.g. nucleic acid amplification reagents, or combinations thereof. The fluid may be the continuous phase and may be an immiscible oil such as fluorocarbon oil, a silicone oil, or a hydrocarbon oil, or a combination thereof. The fluid may advantageously comprise reagents such as surfactants (e.g. octylphenol ethoxylate and/or octylphenoxypolyethoxyethanol), reducing agents (e.g. DTT, beta mercaptoethanol, or combinations thereof).

In practicing methods as described herein, the composition and nature of the partitions, e.g., single-emulsion and multiple-emulsion partitions, may vary. Advantageously, a surfactant may be useful to stabilize the partitions. The PIPs described herein may be prepared as emulsions, e.g., as an aqueous phase fluid dispersed in an immiscible phase carrier fluid (e.g., a fluorocarbon oil, silicone oil, or a hydrocarbon oil) or vice versa. Accordingly, a partition may involve a surfactant stabilized emulsion, e.g., a surfactant stabilized single emulsion or a surfactant stabilized double emulsion. Any convenient surfactant that allows for the desired reactions to be performed in the partitions may be useful. In other aspects, PIPs are not stabilized by surfactants.

Template particles useful in the present invention may further comprise a cell capture moiety. The cell capture moiety acts to capture specific cells, for example, specific types of cells. The capture moiety may comprise an Acrylate-terminated hydrocarbon linker with biotin termination. The capture moiety may be attached to a target-specific capture element, for example aptamers and/or antibodies. Examples of capture moieties and methods thereof are disclosed in PCT application no. PCT/US2019/053426, incorporated herein by reference.

### Reverse Transcription, Amplification, and Sequencing

Methods of the invention generally relate to analysis and sequencing of gene transcripts from single cells modified by RNA guides in genomic areas of interest, for example oncogenes. PCR amplification of products derived from nucleic acid molecules released by single cells can be useful to determine a gene expression profile for a cell for preselected gene mutations, e.g., mutations associated with cancer. For example, identification of a gene or mutation of interest may provide information that the cell from which the nucleic acid molecule was released is expressing gene transcripts associated with cancer as a result of the genomic modification resulting from the RNA guide. Because each nucleic acid molecule is tagged with a barcode unique to the PIP and single cell from which it was released, any gene transcript can be traced back to the PIP and single cell, thereby allowing for the identification of an RNA guide and genotypic modification created by the RNA guide.

For RNA or mRNA sequencing, sequencing may first comprise the step of preparing a cDNA library from barcoded RNA, for example through reverse transcription, and sequencing the cDNA. cDNA sequencing may advantageously allow for the quantification of gene expression within the single cell, and can be useful to identify characteristics of the single cell to, for example, make a diagnosis, prognosis, or determine drug effectiveness. Reverse transcription of cDNA molecules from RNA can be performed both within the PIP or after barcoded RNA molecules have been released from each PIP.

Reverse transcription may be performed using without limitation dNTPs (mix of the nucleotides dATP, dCTP, dGTP and dTTP), buffer/s, detergent/s, or solvent/s, as required, and suitable enzyme such as polymerase or reverse transcriptase. The polymerase used may be a DNA polymerase, and may be selected from Taq DNA polymerase, Phusion polymerase (as provided by Thermo Fisher Scientific, Waltham, Massachusetts ), or Q5 polymerase. Nucleic acid amplification reagents are commercially available, and may be purchased from, for example, New England Biolabs, Ipswich, MA, USA. The reverse transcriptase used in the presently disclosed targeted library preparation method may be for example, maxima reverse transcriptase. In some embodiments, the general parameters of the reverse transcription reaction comprise an incubation of about 15 minutes at 25 degrees and a subsequent incubation of about 90 minutes at 52 degrees.

Reverse transcription may be performed by oligos that have a free, 3' poly-T region. The 3' portions of the cDNA capture oligos may include gene-specific sequences or oligomers, for example capture primers to reverse transcribe RNA guides comprising a capture sequence. The oligomers may be random or "not-so-random" (NSR) oligomers (NSROs), such as random hexamers or NSR hexamers. The oligos may include one or more handles such as primer binding sequences cognate to PCR primers that are used in the amplifying step or the sequences of NGS sequencing adaptors. The reverse transcription primers may include template switching oligos
(TSOs), which may include poly-G sequences that hybridize to and capture poly-C segments added during reverse transcription.

Reverse transcription of non-polyadenylated RNA may comprise use of a capture sequence and a capture primer or probe. Primer sequences may comprise a binding site, for example a primer sequence that would be expected to hybridize to a complementary sequence, if present, on any nucleic acid molecule released from a cell and provide an initiation site for a reaction. The primer sequence may also be a "universal" primer sequence, i.e. a sequence that is complementary to nucleotide sequences that are very common for a particular set of nucleic acid fragments. Primer sequences may be P5 and P7 primers as provided by Illumina, Inc., San Diego, California. The primer sequence may also allow a capture probe to bind to a solid support, such as a template particle.

Reverse transcription can also be useful for adding a barcode unique to the PIP or a UMI, or both to cDNA. This process may comprise hybridizing the reverse transcription primer to the probe followed by a reverse transcription reaction. The complement of a nucleic acid when aligned need not be perfect; stable duplexes may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

Nucleic acid molecules may advantageously be amplified prior to sequencing. Amplification may comprise methods for creating copies of nucleic acids by using thermal cycling to expose reactants to repeated cycles of heating and cooling, and to permit different temperature-dependent reactions (e.g. by Polymerase chain reaction (PCR). Any suitable PCR method known in the art may be used in connection with the presently described methods. Non limiting examples of PCR reactions include real-time PCR, nested PCR, multiplex PCR, quantitative PCR, or touchdown PCR. Notably, each amplified copy of the nucleic acid molecule will comprise the barcode unique to a PIP for identifying the PIP and cell from which the nucleic acid molecule was released and a UMI. Methods for amplification many include whole genome amplification.

Sequencing nucleic acid molecules may be performed by methods known in the art. For example, see, generally, Quail, et al., 2012, A tale of three next generation sequencing platforms: comparison of Ion Torrent, Pacific Biosciences and Illumina MiSeq sequencers, BMC Genomics 13:341. Nucleic acid molecule sequencing techniques include classic dideoxy sequencing reactions (Sanger method) using labeled terminators or primers and gel separation in slab or capillary, or preferably, next generation sequencing methods. For example, sequencing may be performed according to technologies described in U.S. Pub. 2011/0009278, U.S. Pub. 2007/01 14362, U.S. Pub. 2006/0024681, U.S. Pub. 2006/0292611, U.S. Pat. 7,960,120, U.S. Pat. 7,835,871, U.S. Pat. 7,232,656, U.S. Pat. 7,598,035, U.S. Pat. 6,306,597, U.S. Pat. 6,210,891, U.S. Pat. 6,828,100, U.S. Pat. 6,833,246, and U.S. Pat. 6,911,345, each incorporated by reference.

The conventional pipeline for processing sequencing data includes generating FASTQ-format files that contain reads sequenced from a next generation sequencing platform, aligning these reads to an annotated reference genome, and quantifying expression of genes. These steps are routinely performed using known computer algorithms, which a person skilled in the art will recognize can be used for executing steps of the present invention. For example, see Kukurba, Cold Spring Harb Protoc, 2015 (11):951-969, incorporated by reference.

### Tubes for generating PIPs

As described above, tubes may be selected based on the volume of sample from which cells need to be separated and/or based on the number of cells to be separated. For example, the tube may be a single large tube, such as a conical centrifuge tube, such as a Falcon^{®} as sold by Corning Inc., Corning, New York, for example a tube with a volume of less than 40 mL. Such tubes may be advantageous where the number of cells to be analyzed is between 100,000 and 1 million cells or greater than 1 million cells. This method is useful when analyzing cells for targeted coverage of heterogeneous cell types and exploring pathways in complex tissues, for example in cancer detection in mixed cell populations.

The tubes may also be wells, such as standard 96 sample well kit. The well may be part of a microplate with multiple wells each using a tube. The microplate may comprise any number of wells as desired, for example 6-1536 wells. Advantageously, the microplate may comprise 96 wells. Wells may be advantageous where the number of cells to be analyzed is about 100 cells. This method is useful when deep profiling homogenous cells under different conditions, such as early cancer detection in a tumor site.

The tubes may also be centrifuge, microcentrifuge, or PCR tubes, such as those sold by Eppendorf ^{®}, Hamburg, Germany. Such tubes, for example, may be between 1 and 6mL and are advantageous where the number of cells to be analyzed is about 10,000 cells. This method is useful when deep profiling heterogeneous cell populations, for example in early cancer detection in mixed cell populations.

As described above, because cells are encapsulated in PIPs simultaneously, methods of the present invention are easily scaled for the analysis of any number of cells. For example, tubes may be selected to analyze at least 1 million cells, at least 2 million cells, at least 10 million, at least than 100 million cells, or 200 million cells of greater. Additionally, because cells are encapsulated simultaneously, for any tubes and any number of cells sample preparation for sequencing may be completed within one day, and can be completed within three hours. Moreover, preparation of samples within each tube may be completed in as little time as about 5 minutes or about 2 minutes.

Primers and/or reagents may be added to the PIPS after formation of the PIPs in the tube. Primers and/or reagents may be added in one step, or in more than one step. For instance, the primers may be added in two or more steps, three or more steps, four or more steps, or five or more steps. Regardless of whether the primers are added in one step or in more than one step, they may be added after the addition of a lysing agent, prior to the addition of a lysing agent, or concomitantly with the addition of a lysing agent. When added before or after the addition of a lysing agent, PCR primers may be added in a separate step from the addition of a lysing agent.

### Incorporation by Reference

References and citations to other documents, such as patents, patent applications, patent publications, journals, books, papers, web contents, have been made throughout this disclosure. All such documents are hereby incorporated herein by reference in their entirety for all purposes.

### Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.

### Examples

### Example 1

Cells modified by an RNA guided expression system and expressing non-polyadenylated copies of the RNA guides were mixed with template particles and encapsulated near-instantly along with primers for capture of non-polyadenylated RNA guides and reverse transcription of polyadenylated RNA. Non-polyadenylated RNA guides comprise the capture sequence: 5'-GGCTAGTCCGTTATCAACTTGNNNNNNNNNNNNAAAGTCATAAGGCGTCACAAGCAATCACTC-3'
incorporated within the guide. Capture primers for RNA guides and poly-T reverse transcription primers are combined in a 1:20 ratio capture primer to poly-T ratio and also encapsulated in the PIPs during PIP formation. RNA in each PIP is then reverse transcribed and amplified by whole genome amplification.

Partitioning of cells, reverse transcription, and amplification take place in a single day. During Day 2, the cDNA is then purified using bead cleanup, with the smallest fragments in the supernatant representing the cDNA generated from the RNA guides and the precipitate containing cDNA generated from RNA transcripts. During Day 3, cDNA from RNA guides and cDNA from RNA transcripts are enriched in parallel.

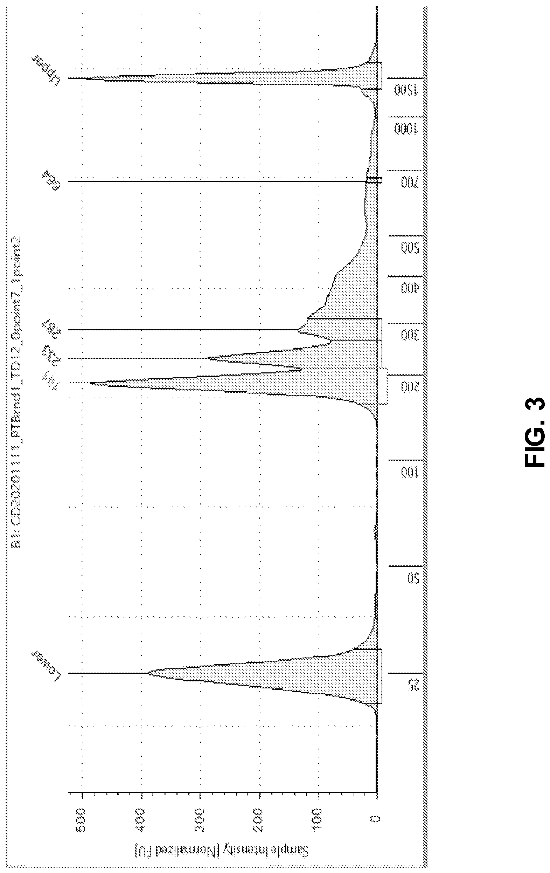

FIG. 3 depicts a graph of post-cleanup cDNA fractions from PIPs, with cDNA associated with the smaller RNA guides shown in the sample by the lower peak and cDNA associated with larger RNA transcripts shown in the sample as larger peaks.

### Clauses

Clause 1. A method for gene expression profiling, the method comprising:
combining a plurality of cells with template particles, wherein cells have been genetically modified by an RNA guided endonuclease and the cells comprise copies of the RNA guide further comprising a capture sequence;
generating a plurality of uniform partitions near-instantly that encapsulate a single one of the template particles and a single one of the cells to form pre-templated instant partitions (PIPs); and
releasing in each PIP nucleic acid molecules from the cells, including at least the RNA guides and RNA transcripts.

Clause 2. The method of clause 1, wherein the RNA guides are non-polyadenylated.

Clause 3. The method of clause 2, the method further comprising reverse transcribing the non-polyadenylated RNA guides and RNA transcripts to form a cDNA complement of the RNA molecules further comprising a unique molecular identifier and a barcode unique to the PIP.

Clause 4. The method of clause 3, wherein the step of reverse transcribing the non-polyadenylated RNA guides comprises hybridizing a capture primer to the capture sequence of the non-polyadenylated RNA guides.

Clause 5. The method of clause 2, wherein in the combining step the RNA guides further comprise a unique molecular identifier.

Clause 6. The method of clause 5, the method further comprising:
reverse transcribing the RNA transcripts to form a cDNA complement of each RNA transcript further comprising a unique molecular identifier and a barcode unique to the PIP; and
reverse transcribing the RNA guides to form a cDNA complement of each non-polyadenylated RNA guide further comprising a barcode unique to the PIP.

Clause 7. The method of clause 3, further comprising sequencing the cDNA molecules to generate sequence reads associated with each RNA guide and sequence reads associated with each RNA transcript.

Clause 8. The method of clause 8, further comprising associating each RNA guide sequence read with each RNA transcript sequence read having the same barcode unique to a PIP.

Clause 9. The method of clause 8, wherein the RNA guided endonuclease and RNA guide modify the expression of RNA transcripts in the cell as compared to a cell in the absence of the RNA guide.

Clause 10. The method of clause 9, wherein the RNA guided endonuclease and RNA guide modify the DNA of the cell to reflect a genotype associated with a genetic disease.

Clause 11. The method of the clause 10, wherein the genetic disease is cancer.

Clause 12. The method of clause 1, wherein the RNA guides comprise a constant region and wherein the capture sequences are in constant regions of the RNA guides.

Clause 13. The method of clause 12, wherein the constant regions are selected from a 2 stem loop region or a 3' end region.

Clause 14. The method of clause 4, wherein the combining step comprises further combining with the plurality of cells and template particles primers for reverse transcription, including at least the capture primers, and the generating step comprises encapsulating the primers into each PIP.

Clause 15. The method of clause 1, wherein the RNA guided endonuclease is a Cas endonuclease.

Clause 16. The method of clause 1, wherein the step of combining template particles comprises:
combining the template particles with the plurality of cells in a first fluid and
adding a second fluid to the first fluid,
the step of generating a plurality of uniform partitions near-instantly comprises:
agitating the fluids to generate the PIPs that contain a single one of the template
   particles and a single one of the single cells; and
the step of releasing in each PIP nucleic acid molecules from the cells comprises:
   lysing each of the single cells contained within the PIPs.

Clause 17. The method of clause 16, wherein the first fluid and the second fluid are immiscible.

Clause 18. The method clause 1, wherein the step of combining cells and template particles and the step of generating a plurality of uniform partitions near-instantly are performed in a tube, wherein the tube is a centrifuge, microcentrifuge, or polymerase chain reaction (PCR) tube.

Clause 19. The method of clause 18, wherein the step of generating in the tube a plurality of uniform partitions near-instantly that encapsulate a single one of the template particles and a single one of the cells comprises generating a plurality of partitions that encapsulate a single one of the template particles and do not encapsulate a cell.

Clause 20. A method for gene expression profiling, the method comprising:
combining a plurality of cells with template particles, wherein cells have been genetically modified by an RNA guided endonuclease and comprise a non-polyadenylated copy of the RNA guide, and a polyadenylated RNA guide barcode;
generating a plurality of uniform partitions near-instantly that encapsulate a single one of the template particles and a single one of the cells to form pre-templated instant partitions (PIPs);
releasing in each PIP nucleic acid molecules from the cells, including at least the polyadenylated RNA guide barcodes and RNA transcripts; and
reverse transcribing the polyadenylated RNA guide barcodes and RNA transcripts to form a cDNA complement of the RNA molecules further comprising a unique molecular identifier and a barcode unique to the PIP.

## Claims

1. A method for gene expression profiling, the method comprising:
combining a plurality of cells with template particles, wherein cells have been genetically modified by an RNA guided endonuclease and comprise a non-polyadenylated copy of the RNA guide, and a polyadenylated RNA guide barcode;
generating a plurality of uniform partitions near-instantly that encapsulate a single one of the template particles and a single one of the cells to form pre-templated instant partitions (PIPs);
releasing in each PIP nucleic acid molecules from the cells, including at least the polyadenylated RNA guide barcodes and RNA transcripts; and
reverse transcribing the polyadenylated RNA guide barcodes and RNA transcripts to form cDNA molecules further comprising a unique molecular identifier and a barcode unique to the PIP.

2. The method of claim 1, wherein the RNA guided endonuclease is a Cas endonuclease.

3. The method of claim 1 or claim 2, wherein the step of combining the plurality of cells with template particles comprises:
combining the template particles with the plurality of cells in a first fluid and
adding a second fluid to the first fluid, wherein the first fluid and the second fluid are immiscible, and wherein the step of generating a plurality of uniform partitions near-instantly comprises agitating the fluids to generate the PIPs.

4. The method of any one of claims 1 to 3, wherein the step of combining the plurality of cells with template particles and the step of generating a plurality of uniform partitions near-instantly are performed in a tube, wherein the tube is a centrifuge, microcentrifuge, or polymerase chain reaction (PCR) tube.

5. The method of any one of claims 1 to 4, further comprising sequencing the cDNA molecules to generate sequence reads associated with each RNA guide and sequence reads associated with each RNA transcript, and/or, further comprising associating each RNA guide sequence read with each RNA transcript sequence read having the same barcode unique to a PIP.

6. A method for gene expression profiling, the method comprising:
combining a plurality of cells with template particles, wherein cells have been genetically modified by an RNA guided endonuclease and the cells comprise copies of the RNA guide further comprising a capture sequence;
generating a plurality of uniform partitions near-instantly that encapsulate a single one of the template particles and a single one of the cells to form pre-templated instant partitions (PIPs);
releasing in each PIP nucleic acid molecules from the cells, including at least the RNA guides and RNA transcripts; and
wherein the step of generating a plurality of uniform partitions near-instantly further comprises generating a plurality of partitions that encapsulate a single one of the template particles and do not encapsulate a cell.

7. The method of claim 6, wherein the RNA guides are non-polyadenylated.

8. The method of claim 7, the method further comprising reverse transcribing the non-polyadenylated RNA guides and RNA transcripts to form a cDNA complement of the RNA molecules further comprising a unique molecular identifier and a barcode unique to the PIP.

9. The method of claim 8, wherein the step of reverse transcribing the non-polyadenylated RNA guides comprises hybridizing a capture primer to the capture sequence of the non-polyadenylated RNA guides.

10. The method of any one of claims 6 to 9, wherein the RNA guided endonuclease is a Cas endonuclease.

11. The method of any one of claims 6 to 10, wherein the step of combining the plurality of cells with template particles comprises:
combining the template particles with the plurality of cells in a first fluid and
adding a second fluid to the first fluid,
wherein the first fluid and the second fluid are immiscible, and
wherein the step of generating a plurality of uniform partitions near-instantly comprises agitating the fluids to generate the PIPs.

12. The method of any one of claims 6 to 11, wherein the step of combining the plurality of cells with template particles and the step of generating a plurality of uniform partitions near-instantly are performed in a tube, wherein the tube is a centrifuge, microcentrifuge, or polymerase chain reaction (PCR) tube.

13. The method of claim 8 or 9, further comprising sequencing the cDNA molecules to generate sequence reads associated with each RNA guide and sequence reads associated with each RNA transcript, and/or, further comprising associating each RNA guide sequence read with each RNA transcript sequence read having the same barcode unique to a PIP.

14. A system comprising at least one processor and a non-transitory computer readable medium comprising instructions that, when executed by the at least one processor, cause the system to perform a method according to any one of claims 1-13.

15. A non-transitory computer readable storage medium comprising instructions that, when executed by at least one processor, cause a computing device to perform a method according to any one of claims 1-13.
